# NOUVEAU FASCICULE DE BREVET EUROPEEN

(11) **EP 0 645 081 B2**
(45) Date de publication et mention de la décision concernant l'opposition: **24.01.2007**
(45) Mention de la délivrance du brevet: 25.07.2001
(21) Numéro de dépôt: 94420260.5
(22) Date de dépôt: 28.09.1994
(51) Int. Cl.: A01M 1/20

(54) **Dispositif pour effectuer des traitements insecticides, et leur utilisation dans les habitations**
Vorrichtung zur insektiziden Behandlung und deren Verwendung in Wohnungen
Device for effecting insecticidal treatments, and their use in houses

(30) Priorité: 28.09.1993 FR 9311762
(43) Date de publication de la demande: 29.03.1995
(73) Titulaire: OMS INVESTMENTS, Inc., Wilmington, DE 19899-8985 (US)
(72) Inventeur: Karg, Jörn E., D-67661 Kaiserslautern (DE)
(74) Mandataire: Bentham, Stephen

(56) Documents cités:
- EP-A- 0 019 010
- FR-A- 1 601 251
- FR-A- 2 075 314
- FR-A- 2 663 507
- US-A- 2 809 863
- US-A- 3 424 380
- US-A- 4 157 787
- US-A- 4 158 440
- US-A- 4 845 131
- US-A- 4 849 606
- DATABASE WPI Week 9047, Derwent Publications Ltd., London, GB; AN 90-351233 & JP-A-2 252 462 (NIPPON PETROCHEM) & PATENT ABSTRACTS OF JAPAN vol. 14, no. 578 (C-791) 21 Décembre 1990 & JP-A-02 252 462 (NIPPON PETROCHEM)

## Description

La présente invention est relative à un nouveau dispositif utile pour les traitements insecticides et/ou répulsifs à l'égard des insectes dans les domaine des habitations humaines, et plus particulièrement utile pour les traitements des espaces confinés contre les mites.

Le dispositif selon l'invention est caractérisé en ce qu'il comprend:
* un récipient en matière plastique transparente en forme d'une cuvette dont la face ouverte a un rebord plat,
* une feuille (ou membrane) en matière plastique imperméable aux liquides et perméables aux vapeurs d'huiles essentielles à température ambiante, cette feuille étant fixée au rebord plat du récipient transparent et adhérant à ce récipient transparent d'une manière à ne pas pouvoir être saisie à la main et/ ou être détachée du rebord plat en tirant à la main,
* une feuille protectrice de couverture en matière plastique collant également disposée sur la face du récipient transparent munie de rebords plats, cette feuille adhérant à la précédente feuille perméable par collage et étant susceptible d'être saisie à la main et/ou être détachée du rebord plat en tirant à la main,
* une composition insecticide, et/ou répulsive à l'égard des insectes, cette composition étant liquide capable de s'évaporer totalement à température ambiante et avant une activité insecticide et/ou répulsive à l'état de vapeur,
et comprenant une huile essentielle ainsi qu'un adjuvant de diffusion, la quantité d'huile essentielle étant de 20 à 85°, et la quantité d'adjuvant de diffusion étant de 15% à 80%,
* éventuellement des moyens d'accrochage et/ou de suspension du dispositif et/ou du récipient transparent,
* un emballage en carton replié en deux, de manière à contenir en son intérieur les bords plats et les deux feuilles, perméables et protectrices, la cuvette transparente étant visible et transparente en dehors de cet emballage carton.

Les documents EP 0019100 (récipient en forme de goutte) et US 4157787 (récipient en forme de cuvette) divulguent des tels dispositifs, mais aucun de ces documents pris seul ou en combinaison ne permet de retrouver toutes les caractéristiques propre à notre invention, et notamment la combinaison du récipient et du suremballage carton.

Un exemple de réalisation du dispositif selon l'invention est représenté sur les figures.

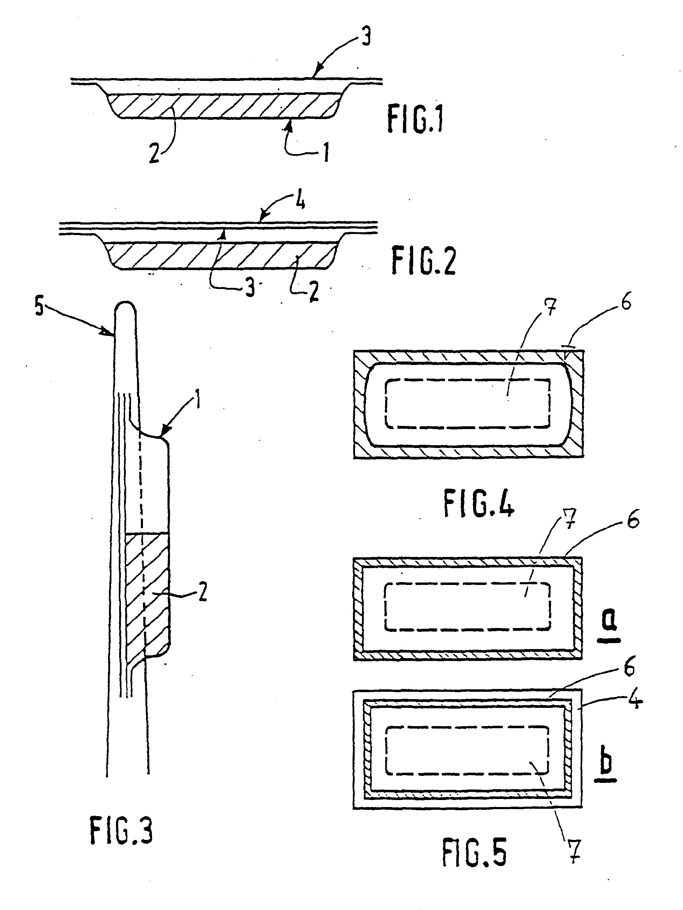

La figure (1) représente le dispositif de l'invention dans son état de fonctionnement insecticide et/ou répulsif, sauf que l'emballage de carton n'a pas été représenté.

La figure (2) représente le dispositif de l'invention dans son état convenant pour le stockage, sauf que l'emballage de carton n'a pas été représenté.

La figure (3) représente le dispositif de l'invention dans son emballage, dans un état convenant pour le stockage et la vente et l'utilisation.

En figure (1) la cuvette transparente contient le liquide (2) et est couverte par la membrane perméable (3). Outre la composition insecticide et/ou répulsive, la cuvette, ou récipient transparent peut aussi contenir une petite quantité d'air, par exemple 1 à 10 % en volume.

La figure (4) représente le dispositif de l'invention la feuille protectrice recouvrant le dispositif précédent.

La figure (5) représente le dispositif de l'invention l'emballage en carton contenant le dispositif de la figure (2) de manière à ce que les rebords soient à l'intérieur. L'emballage carton peut jouer le rôle de présentoir publicitaire. Selon une variante de l'invention, cet emballage de carton comprend un évidement ou une partie détachable, permettant de retirer la pellicule protectrice sans détacher le récipient transparent de cet emballage carton. Cette variante de l'invention est particulièrement avantageuse en ce qu'elle permet de laisser visible sur l'emballage du dispositif de l'invention en cours d'utilisation et de fonctionnement les indications relatives au mode d'emploi et à la nature du contenu du dispositif de l'invention. De telles indications sont utiles dans un but de sécurité de l'utilisation.

Le dispositif selon l'invention est particulièrement adapté pour les espaces confinés, notamment les espaces clos de faible volume comme l'intérieur des meubles, armoires et placards. Bien qu'il puisse être utilisé contre différents types d'insectes, le dispositif de l'invention est particulièrement avantageux contre les mites. Le dispositif selon l'invention est donc particulièrement avantageux pour la protection des textiles dans des espaces confinés, notamment la protection des vêtements dans les meubles de lieux d'habitation tels que les appartements ou les maisons.

Une utilisation particulière consiste en ce qu'un premier dispositif selon l'invention est mis en oeuvre, puis, quand celui-ci est vide, un deuxième est mis en oeuvre à la suite sans période d'interruption de la protection entre ces deux mises en oeuvre.

Un autre avantage du dispositif de l'invention est de permettre de choisir facilement la durée d'utilisation (par exemple 1 mois, ou 2 mois, ou 6 mois ou autre) lors de la fabrication du dit dispositif. Cette durée peut ainsi être modifiée de façon simple.

Un avantage du dispositif selon l'invention est d'être efficace pendant une longue durée. En outre, lorsque le produit insecticide et/ou répulsif est épuisé, l'utilisateur a la possibilité de manière très simple de voir et constater que le récipient transparent est vide et doit être remplacé. En conséquence, l'activité insecticide et/ou répulsive est maintenue constamment et il n'y a pas de période transitoire sans insecticide/répulsif, ce qui est capital pour ne pas laisser des périodes sans insecticide/répulsif au cours desquelles les insectes peuvent à nouveau se reproduire et se développer et causer des dégâts.

Dans le ces des mites, l'invention est particulièrement avantageuse en ce qu'elle permet d'éviter d'endommager les textiles, notamment les vêtements, pendant des périodes transitoires sans matière active.

Les deux feuilles perméable et protectrice peuvent adhérer au récipient transparent par collage ou par soudure. D'une manière ou d'une autre, on fait en sorte que l'adhésion de la feuille perméable est bien plus forte que l'adhésion de la feuille protectrice, en sorte que la feuille protectrice peut être détachée par traction à la main, tandis que la feuille perméable ne peut pas être détachée par traction à la main. La différence d'adhésion peut être obtenue soit par la taille de la surface sur laquelle il y a adhésion (et c'est le moyen préféré), soit par la nature de l'adhésif.

La figure (4) représente une vue de dessus du dispositif selon la figure (1). La partie hachurée (6) montre la surface où l'adhésion a lieu entre le récipient transparent (1) et la membrane perméable (3). La zone (7) correspond au liquide (2) vu de dessus.

La figure (5) représente une vue de dessus du dispositif selon la figure (2). La partie hachurée montre la surface (6) où l'adhésion a lieu entre le récipient transparent (1) et la membrane perméable (3). Deux représentations ont été faites, selon les figures (5a) et (5b) selon que la feuille de protection (4) dépasse, ou non, de la membrane perméable (3).

Les compositions liquides insecticides et/ou répulsives sont des compositions comprenant de 20 à 85 % d'huiles essentielles, de préférence de 40 à 75 %, ainsi que 15 à 80 % d'un agent d'évaporation (ou adjuvant de diffusion), de préférence de 25 à 60 %.

Comme huile essentielle, on peut utiliser l'essence de bois de cèdre, l'essence de feuilles de cèdre, l'essence de thuya, l'essence de lavande, l'essence de lavandin, l'essence de citronnier, l'essence de Litsea cubeba, l'essence de Verty. De préférence l'huile essentielle mise en oeuvre dans l'invention est l'essence de bois de cèdre ou l'essence de lavande.

L'agent d'évaporation (ou adjuvant de diffusion) mis en oeuvre dans l'invention peut être l'un des constituants de l'huile essentielle. Il est de préférence un agent liquide ayant une vitesse d'évaporation au moins deux fois supérieure à la vitesse d'évaporation de la matière active principale, et de préférence plus de trois fois supérieure à la vitesse d'évaporation de la matière active principale. Cet agent d'évaporation est avantageusement miscible avec la matière active principale.

Plus particulièrement, cet agent d'évaporation (ou adjuvant de diffusion) est de préférence choisi dans le groupe constitué par l'acétate d'hexyle (avantageusement de n-hexyle), l'acétate d'isobutyle, l'acétate de cyclohexyle, l'acétate de prényle, l'acétate d'isonoyle, l'acétate d'isobomyle, l'acétate de linalyle, l'acétate de benzyle, le butyrate d'éthyle, le camphre, le caproate d'éthyle, le caproate de méthyle, le carène, le limonène (ou essence terpénique d'oranger), l'heptanoate d'allyle, l'hèxènoate de méthyle, l'isobutyrate d'éthyle, la méthylpentylcétone, le myrcène, le phaellandrène, le pinène, le valérianate de butyle, le terpinolène et les huiles paraffiniques (hydrocarbures saturés liquides à température ambiante). Les agents d'évaporation peuvent être utilisés seuls ou en mélanges. Les adjuvants de diffusion qui viennent d'être nommés sont remarquables par leur efficacité en conjonction avec les huiles essentielles.

Les compositions liquides insecticides et/ou répulsives selon l'invention peuvent contenir en outre un colorant, en quantité de préférence inférieure à 1 %. Elles peuvent aussi contenir divers composés aromatiques tels que des produits dérivés de la vanilline, notamment l'éthylvanilline, la coumarine, l'essence de bois de santal, l'acétate de terpinyle, le citral, les delphènes, l'essence de terpentine. Ces composes peuvent former de 2 à 40 % des compositions de l'invention.

Les récipients transparents sont avantageusement en matière polymérique telles que le polyéthylène ou le polypropylène ou leurs copolymères. L'épaisseur du matériau est suffisante pour empêcher toute perméation tant du liquide que de sa vapeur. Le volume utile du récipient est généralement compris entre 2 et 120 cm³.

La nature et l'épaisseur de la membrane ou feuille perméable sont choisies de manière à présenter les caractéristiques de perméabilité indiquées plus haut. De manière avantageuse, on choisit des membranes polymériques satisfaisant au test suivant : un récipient plein de 10 grammes d'essence de bois de cèdre est pourvu d'une face libre appliquée contre une membrane verticale sur une surface de 14 cm². La membrane doit être telle que, au bout de trois mois, 0,8 g de l'essence a disparu.

Des membranes particulièrement adaptées à l'objet de l'invention sont les membranes en matière polymérique telle que le polyéthylène à haute densité avec une épaisseur de 50 à 300 microns. La surface utile pour la perméation est généralement comprise entre 5 et 35 cm².

Les feuilles ou membranes protectrices sont avantageusement en matière polymérique telles que le polypropylène. Elles sont pourvues d'un agent collant capable de ne pas endommager la membrane perméable lorsque la feuille protectrice est détachée de cette membrane.

L'exemple suivant, donné à titre non limitatif, illustre l'invention et montre comment elle peut être mise en oeuvre. A défaut d'indication spécifique contraire, tous les pourcentages indiqués sont des pourcentages pondéraux.

### EXEMPLE 1

Un récipient en forme de cuvette de 8 mm de profondeur contient 10 g d'un mélange comprenant de l'essence de bois de cèdre, de l'essence de feuilles de cèdre, de l'acétate d'isobornyle (adjuvant de diffusion), de l'essence terpénique d'oranger (adjuvant de diffusion), de l'essence de terpentine, du phaellandrène (adjuvant de diffusion), de l'acétate de benzyle (adjuvant de diffusion). Tous les adjuvants de cette composition ont une vitesse d'évaporation supérieure à plus de 3 fois à celle de l'essence de bois de cèdre.

Ce récipient est constitué de polypropylène semi-rigide et transparent de 0,5 mm d'épaisseur. Sur le rebord plat de ce récipient transparent se trouve une membrane perméable de polyéthylène de haute densité de 200 microns dont la surface utile pour la perméation (égale à la surface d'ouverture du récipient transparent) est de 14 cm².

Le dispositif est réalisé selon une forme telle que décrite dans les dessins joints à la présente description.

La feuille protectrice est en polypropylène de 280 microns est munie d'un adhésif. Un coin de cette couche protectrice est laissé sans adhésif de manière à ce que cette couche puisse être saisie à la main.

Ce dispositif est suspendu dans un espace confiné de 1 m³ pendant 3 mois. A la fin des 3 mois, 9 g. du mélange ont disparus et cela se voit. Ce résultat est à comparer au même test effectué avec de l'essence de bois de cèdre sans adjuvant de diffusion : la quantité disparue est de 0,8 g. au lieu de 9 g.

Au début des trois mois, on suspend des vêtements dans l'espace confiné, puis on suspend le dispositif tel que décrit ci-dessus, puis on dépose des larves de mites à 3 endroits distincts des vêtements suspendus.

A la fin des 3 mois, on observe que
* il n'y a aucun dommage causé aux vêtements,
* les larves sont mortes
* il n'y a aucune chrysalide ni aucune autre forme de l'insecte à aucune forme de développement du cycle de la vie des mites.

### EXEMPLE 2

On répète l'exemple 1 avec la composition suivante.

10 % d'essence de lavande, 33 % d'essence de lavandin, 22 % d'acétate de linalyle (adjuvant de diffusion), 30 % de limonène, 5 % d'acétate d'isononyle (adjuvant de diffusion). On obtient des résultats semblables à ceux de l'exemple 1.

### EXEMPLE 3

On répète l'exemple 1 avec la composition suivante.

5 % de métadelphène, 20 % d'essence de citronnier, 25 % de citral, 10 % d'huile paraffinique (adjuvant de diffusion), 5 % de limonène (adjuvant de diffusion), 35 % d'acétate de terpinyle. On obtient des résultats semblables à ceux de l'exemple 1.

### EXEMPLE 4

On répète l'exemple 1 avec la composition suivante : 40 % d'essence de bois de cèdre, 5 % d'essence de feuilles de cèdre, 20 % d'acétate d'isononyle (adjuvant de diffusion), 2 % d'éthylvanilline, 2 % de coumarine, 15 % de phaellandrène (adjuvant de diffusion), 16 % d'essence de bois de santal. On obtient des résultats semblables à ceux de l'exemple 1.

## Revendications

1. Dispositif utilisable dans la lutte contre les insectes comprenant:
* un récipient (1) en matière plastique transparente en forme d'une cuvette dont la face ouverte a un rebord plat,
* une feuille (3) en matière plastique imperméable aux liquides et perméable aux vapeurs d'huiles essentielles à température ambiante, cette feuille étant fixée au rebord plat du récipient transparent et adhérant à ce récipient transparent d'une manière à ne pas pouvoir être saisie à la main ou être détachée du rebord plat en tirant à la main,
* une feuille (4) protectrice de couverture en matière plastique collant également disposée sur la face du récipient transparent munie de rebords plats, cette feuille adhérant à la précédente feuille perméable par collage et étant susceptible d'être saisie à la main ou être détachée du rebord plat en tirant à la main,
* une composition (2) insecticide, et/ou répulsive à l'égard des insectes, cette composition étant liquide capable de s'évaporer totalement à température ambiante et ayant une activité insecticide et/ou répulsive à l'état de vapeur et comprenant une huile essentielle ainsi qu'un adjuvant de diffusion, la quantité d'huile essentielle étant de 20 à 85% et la quantité d'adjuvant de diffusion étant de 15% à 80%,
* éventuellement des moyens d'accrochage et/ou de suspension du dispositif et/ou du récipient transparent,
* un emballage en carton (5) replié en deux, de manière à contenir en son intérieur les bords plats et les deux feuilles, perméables et protectrices, la cuvette transparente étant visible et transparente en dehors de cet emballage carton.

2. Dispositif selon la revendication 1 **caractérisé en ce que** la composition est active pour détruire ou repousser les mites.

3. Dispositif selon l'une des revendications précédentes **caractérisé en ce que** l'emballage de carton comprend un évidement ou une partie détachable, permettant de retirer la pellicule protectrice sans détacher le récipient transparent de cet emballage carton.

4. Dispositif selon l'une des revendications précédentes **caractérisé en ce que** les compositions liquides insecticides et/ou répulsives sont des compositions comprenant de 20 à 85% d'huiles essentielles, de 15 à 80% d'un adjuvant de diffusion, et éventuellement de 2 à 40% d'un composé aromatique.

5. Dispositif selon l'une des revendications précédentes **caractérisé en ce que** les compositions liquides insecticides et/ou répulsives sont des compositions comprenant de 40 à 75% d'huiles essentielles.

6. Dispositif selon l'une des revendications précédentes **caractérisé en ce que** les compositions liquides insecticides et/ou répulsives sont des compositions comprenant de 25 à 60% d'un adjuvant de diffusion.

7. Dispositif selon l'une des revendications précédentes **caractérisé en ce que** l'huile essentielle est choisie dans le groupe constitué par l'essence de bois de cèdre, l'essence de feuilles de cèdre, l'essence de thuya, l'essence de lavande, l'essence de lavandin, l'essence de citronnier, l'essence de Litsea cubeba, l'essence de Verty.

8. Dispositif selon l'une des revendications précédentes **caractérisé en ce que** l'huile essentielle est choisie dans le groupe constitué par l'essence de bois de cèdre et l'essence de lavande.

9. Dispositif selon l'une des revendications précédentes **caractérisé en ce que** l'adjuvant de diffusion est un agent liquide miscible avec la matière active principale et ayant une vitesse d'évaporation au moins deux fois supérieure à la vitesse d'évaporation de la matière active principale, et de préférence plus de trois fois supérieure à la vitesse d'évaporation de la matière active principale.

10. Dispositif selon l'une des revendications précédentes **caractérisé en ce que** l'adjuvant de diffusion est choisi dans le groupe constitué par l'acétate d'hexyle, l'acétate d'isobutyle, l'acétate de cyclohexyle, l'acétate de prényle, l'acétate de d'isonoyle, l'acétate de d'isobornyle, l'acétate de linalyle, l'acétate de benzyle, le butyrate d'éthyle, le camphre, le caproate d'éthyle, le caproate de méthyle, le carène, le limonène, l'essence terpénique d'oranger, l'heptanoate d'allyle, l'héxènoate de méthyle, l'isobutyrate d'éthyle, la méthylpentylcétone, la myrcène, le phaellandrène, le pinène, le valérianate de butyle, le terpinolène et les huiles paraffiniques, seuls ou en mélanges.

11. Dispositif selon l'une des revendications précédentes **caractérisé en ce que** le volume utile du récipient est compris entre 2 et 120 cm³.

12. Dispositif selon l'une des revendications précédentes **caractérisé en ce que** la membrane (3) est une membrane polymérique satisfaisant au test suivant: un récipient plein de 10 grammes d'essence de bois ce cèdre est pourvu d'une face libre appliquée contre une membrane verticale sur une surface de 14 cm²; la membrane doit être telle que, au bout de trois mois, 0,8 g de l'essence a disparu.

13. Dispositif selon l'une des revendications précédentes caratérisé en ce que la l'épaisseur de la membrane (3) perméable est 50 à 300 microns.

14. Dispositif selon l'une des revendications précédentes **caractérisé en ce que** la surface utile pour la perméation est comprise entre 5 et 35 cm².

15. Dispositif selon l'une des revendications précédentes **caractérisé en ce que** la membrane perméable (3) est en polyéthylène et/ou que la feuille protectrice est en polypropylène.

16. Utilisation du dispositif selon l'une des revendications précédentes pour la protection des textiles dans des espaces confinés, notamment la protection des vêtements dans les meubles de lieux d'habitation.

17. Utilisation selon la revendication 15 **caractérisé en ce qu'**un premier dispositif selon l'invention est mis en oeuvre, puis que, quand celui-ci est vide, un deuxième est mis en oeuvre à la suite sans période d'interruption de la protection entre ces deux mises en oeuvre.

## Claims

1. Device usable for combating insects, comprising:
- a transparent plastic container (1) in the form of a dish whose open face has a flat flange,
- a plastic sheet (3) impervious to liquids and permeable to the vapours of essential oils at ambient temperature, this sheet being attached to the flat flange of the transparent container and adhering to this transparent container so as not to be capable of being grasped manually or detached from the flat flange by pulling manually,
- a protective cover sheet (4) made of adhesive plastic, also arranged on the face of the transparent container which is provided with flat flanges, this sheet adhering to the above permeable sheet by adhesive bonding and being capable of being grasped manually or being detached from the flat flange by pulling manually,
- an insecticidal and/or insect-repellent composition (2), this composition being liquid capable of evaporating completely at ambient temperature and having an insecticidal and/or repellent activity in the vapour state and comprising an essential oil as well as a diffusion adjuvant, the amount of essential oil being from 20 to 85% and the amount of diffusion adjuvant being from 15% to 80%,
- optionally means for attaching and/or hanging the device and/or the transparent container,
- a cardboard package (5) folded in two, so as to contain within it the flat edges and the two, permeable and protective, sheets, the transparent dish being visible and transparent outside this cardboard package.

2. Device according to Claim 1, **characterized in that** the composition is active in destroying or repelling clothes moths.

3. Device according to either of the preceding claims, **characterized in that** the cardboard package comprises a cavity or a detachable part allowing the protective film to be pulled off without detaching the transparent container from this cardboard package.

4. Device according to one of the preceding claims, **characterized in that** the insecticidal and/or repellent liquid compositions are compositions comprising from 20 to 85% of essential oils, from 15 to 80% of a diffusion adjuvant, and optionally from 2 to 40% of an aromatic compound.

5. Device according to one of the preceding claims, **characterized in that** the insecticidal and/or repellent liquid compositions are compositions comprising from 40 to 75% of essential oils.

6. Device according to one of the preceding claims, **characterized in that** the insecticidal and/or repellent liquid compositions are compositions comprising from 25 to 60% of a diffusion adjuvant.

7. Device according to one of the preceding claims, **characterized in that** the essential oil is chosen from the group consisting of cedar wood essence, cedar foliage essence, thuya essence, lavender essence, lavandin essence, lemon tree essence, Litsea cubeba essence, Verty essence.

8. Device according to one of the preceding claims, **characterized in that** the essential oil is chosen from the group consisting of cedar wood essence and lavender essence.

9. Device according to one of the preceding claims, **characterized in that** the diffusion adjuvant is a liquid agent miscible with the main active substance and having a rate of evaporation at least two times higher than the rate of evaporation of the main active substance, and preferably more than three times higher than the rate of evaporation of the main active substance.

10. Device according to one of the preceding claims, **characterized in that** the diffusion adjuvant is chosen from the group consisting of hexyl acetate, isobutyl acetate, cyclohexyl acetate, prenyl acetate, isonoyl acetate, isobornyl acetate, linalyl acetate, benzyl acetate, ethyl butyrate, camphor, ethyl caproate, methyl caproate, carina, limonene, orange tree terpene essence, allyl heptanoate, methyl hexenoate, ethyl isobutyrate, methyl pentyl ketone, myrcene, phellandrene, pinene, butyl valerate, terpinolene and paraffin oils, alone or as mixtures.

11. Device according to one of the preceding claims, **characterized in that** the effective volume of the container is between 2 and 120 cm³.

12. Device according to one of the preceding claims, **characterized in that** the membrane (3) is a polymeric membrane satisfying the following test: a full container of 10 grams of cedar wood essence is provided with a free face applied against a vertical membrane over an area of 14 cm²; the membrane should be such that, after three months, 0.8 g of the essence has disappeared.

13. Device according to one of the preceding claims, **characterized in that** the thickness of the permeable membrane (3) is 50 to 300 microns.

14. Device according to one of the preceding claims, **characterized in that** the effective area for permeation is between 5 and 35 cm².

15. Device according to one of the preceding claims, **characterized in that** the permeable membrane (3) is made of polyethylene and/or that the protective sheet is made of polypropylene.

16. Use of the device according to one of the preceding claims for the protection of textiles in confined spaces, especially the protection of garments in furniture in dwellings.

17. Use according to Claim 15, **characterized in that** a first device according to the invention is used and then that, when the latter is empty, the second one is used to follow without any period of interruption of the protection between these two uses.

## Patentansprüche

1. Für die Bekämpfung von Insekten verwendbare Vorrichtung, die umfaßt:
- einen Behälter (1) aus einem durchsichtigen Kunststoffmaterial in Form einer Wanne, deren offene Seite eine flache Umrandung aufweist,
- eine Folie (3) aus einem Kunststoffmaterial, die bei Umgebungstemperatur für Flüssigkeiten undurchlässig und für etherische Öldämpfe durchlässig ist, wobei diese Folie an der flachen Umrandung des durchsichtigen Behälters befestigt ist und so fest an dem durchsichtigen Behälter haftet, dass sie nicht mit der Hand ergriffen oder durch Ziehen mit der Hand von der flachen Umrandung abgelöst werden kann,
- eine abdeckende Schutzfolie (4) aus einem klebenden Kunststoffmaterial, die ebenfalls auf der Seite des durchsichtigen Behälters, die mit flachen Umrandungen versehen ist, angeordnet ist, wobei diese Folie auf der vorgenannten, durchlässigen Folie durch Verklebung haftet und von Hand ergriffen oder durch Ziehen mit der Hand von der flachen Umrandung abgelöst werden kann,
- eine isektizide und/oder auf Insekten abwehrend bzw. vertreibend wirkende Zusammensetzung (2), wobei diese Zusammensetzung flüssig ist und bei Umgebungstemperatur vollständig zu verdampfen vermag, die im Dampfzustand eine insektizide und/oder Insekten abwehrende bzw. vertreibende Wirkung hat und ein etherisches Öl sowie einen die Verbreitung fördernden Hilfsstoff enthält, wobei die Menge an etherischem Öl 20 bis 85 % und die Menge an die Verbreitung förderndem Hilfsstoff 15 % bis 80 % beträgt,
- gegebenenfalls Mittel zum Verhaken und/oder zum Aufhängen der Vorrichtung und/oder des durchsichtigen Behälters,
- eine Verpackung (5) aus Pappe, die so aufeinander gefaltet ist, dass sie in ihrem Inneren die flachen Umrandungen und die beiden Folien, d. h. die durchlässige Folie und die Schutzfolie, aufnimmt, wobei die durchsichtige Wanne außerhalb dieser Pappverpackung sichtbar und durchsichtig ist.

2. Vorrichtung nach Anspruch 1, **dadurch gekennzeichnet, dass** die Zusammensetzung so wirkt, dass Motten vernichtet oder abgewehrt bzw. vertrieben werden.

3. Vorrichtung nach einem der vorhergehenden Ansprüche, **dadurch gekennzeichnet, dass** die Pappverpackung eine Aussparung oder einen ablösbaren Teil aufweist, die/der es ermöglicht, die Schutzfolie abzuziehen, ohne den durchsichtigen Behälter von dieser Pappverpackung zu lösen.

4. Vorrichtung nach einem der vorhergehenden Ansprüche, **dadurch gekennzeichnet, dass** es sich bei den insektiziden oder Insekten abwehrenden bzw. vertreibenden flüssigen Zusammensetzungen um Zusammensetzungen handelt, die 20 bis 85 % etherische Öle, 15 bis 80 % eines die Verbreitung fördernden Hilfsstoffs und gegebenenfalls 2 bis 40 % einer aromatischen Verbindung enthalten.

5. Vorrichtung nach einem der vorhergehenden Ansprüche, **dadurch gekennzeichnet, dass** es sich bei den insektiziden oder Insekten abwehrenden bzw. vertreibenden flüssigen Zusammensetzungen um Zusammensetzungen handelt, die 40 bis 75 % etherische Öle enthalten.

6. Vorrichtung nach einem der vorhergehenden Ansprüche, **dadurch gekennzeichnet, dass** es sich bei den insektiziden oder Insekten abwehrenden bzw. vertreibenden flüssigen Zusammensetzungen um Zusammensetzungen handelt, die 25 bis 60 % eines die Verbreitung fördernden Hilfsstoffs enthalten.

7. Vorrichtung nach einem der vorhergehenden Ansprüche, **dadurch gekennzeichnet, dass** das etherische Öl unter Zedernholzöl, Zedernblätteröl, Thujaöl, Lavendelöl, Lavandinöl, Citronenöl, *Litsea cubeba-*Öl (May-Chang-Öl), Verty-Öl ausgewählt ist.

8. Vorrichtung nach einem der vorhergehenden Ansprüche, **dadurch gekennzeichnet, dass** das etherische Öl unter Zedernholzöl und Lavendelöl ausgewählt ist.

9. Vorrichtung nach einem der vorhergehenden Ansprüche, **dadurch gekennzeichnet, dass** der die Verbreitung fördernde Hilfsstoff eine mit dem Hauptwirkstoff mischbare Flüssigkeit ist und eine Verdampfungsgeschwindigkeit aufweist, die mindestens zweimal höher ist als die Verdampfungsgeschwindigkeit des Hauptwirkstoffs, vorzugsweise über dreimal höher als die Verdampfungsgeschwindigkeit des Hauptwirkstoffs.

10. Vorrichtung nach einem der vorhergehenden Ansprüche, **dadurch gekennzeichnet, dass** der die Verbreitung fördernde Hilfsstoff unter Hexylacetat, Isobutylacetat, Cyclohexylacetat, Prenylacetat, Insononylacetat, Isobornylacetat, Linalylacetat, Benzylacetat, Ethylbutyrat, Campher, Ethylcaproat, Methylcaproat, Carenen, Limonen, dem Terpenöl aus Orangen, Allylheptanoat, Methylhexenoat, Ethylisobutyrat, Methylpentylketon, Myrcen, Phellandren, Pinen, Butylvalerianat, Terpinolen und Paraffinölen, einzeln oder in Form von Gemischen, ausgewählt ist.

11. Vorrichtung nach einem der vorhergehenden Ansprüche, **dadurch gekennzeichnet, dass** das Nutzvolumen des Behälters im Bereich von 2 bis 120 cm³ liegt.

12. Vorrichtung nach einem der vorhergehenden Ansprüche, **dadurch gekennzeichnet, dass** die Membran (3) eine Polymermembran ist, die den folgenden Test besteht: ein mit 10 g Zedernholzöl gefüllter Behälter ist mit einer freien Seite versehen, die auf einer Fläche von 14 cm² gegen eine vertikal angeordnete Membran gedrückt wird; die Membran muß derart sein, dass nach 3 Monaten 0,8 g des etherischen Öls verschwunden sind.

13. Vorrichtung nach einem der vorhergehenden Ansprüche, **dadurch gekennzeichnet, dass** die Dicke der durchlässigen Membran (3) 50 bis 300 µm beträgt.

14. Vorrichtung nach einem der vorhergehenden Ansprüche, **dadurch gekennzeichnet, dass** die für die Permeation nutzbare Fläche 5 bis 35 cm² beträgt.

15. Vorrichtung nach einem der vorhergehenden Ansprüche, **dadurch gekennzeichnet, dass** die durchlässige Membran (3) aus Polyethylen und/oder dass die Schutzfolie aus Polypropylen besteht.

16. Verwendung der Vorrichtung nach einem der vorhergehenden Ansprüche für den Schutz von Textilien in geschlossenen Räumen, insbesondere für den Schutz von Kleidung in Wohnungsmöbeln.

17. Verwendung nach Anspruch 15, **dadurch gekennzeichnet, dass** zunächst eine erste erfindungsgemäße Vorrichtung eingesetzt wird, wonach, sobald diese erste Vorrichtung leer ist, sich daran anschließend ohne zwischenzeitliche Unterbrechung des Schutzes zwischen diesen beiden Anwendungen eine zweite erfindungsgemäße Vorrichtung eingesetzt wird.
